# EUROPEAN PATENT SPECIFICATION

(11) **EP 4 243 792 B1**
(45) Date of publication and mention of the grant of the patent: **16.10.2024**
(21) Application number: 21816189.1
(22) Date of filing: 09.11.2021
(51) Int. Cl.: A61K 31/00, A61K 31/015, A61K 31/095, A61K 31/136, A61K 31/198, A61K 31/202, A61K 31/205, A61K 31/337, A61K 31/355, A61K 31/375, A61K 31/475, A61K 31/519, A61K 31/655, A61K 31/7048, A61K 33/243, A61K 33/30, A61K 38/00, A61K 45/06, A61P 15/08

(54) **OMEGA-3 POLYUNSATURATED FATTY ACIDS FOR TREATING / PREVENTING MALE INFERTILITY IN PREPUBERTAL CANDIDATES FOR GONADOTOXIC THERAPIES**
MEHRFACH UNGESÄTTIGTE OMEGA-3-FETTSÄUREN ZUR BEHANDLUNG/VERHINDERUNG DER MÄNNLICHEN UNFRUCHTBARKEIT IN PUBERTÄREN KANDIDATEN FÜR GONADOTOXISCHE THERAPIEN
ACIDES GRAS POLYINSATURÉS OMÉGA-3 POUR LE TRAITEMENT OU LA PRÉVENTION DE L'INFERTILITÉ MASCULINE CHEZ DES CANDIDATS PRÉPUBAIRES POUR DES THÉRAPIES GONADOTOXIQUES

(30) Priority: 10.11.2020 IT 202000026798
(43) Date of publication of application: 20.09.2023
(73) Proprietor: Università degli Studi di Perugia, 06123 Perugia (IT)
(72) Inventor: LUCA, Giovanni, 06123 Perugia (PG) (IT); CALAFIORE, Riccardo, 06123 Perugia (PG) (IT); VECCHINI, Alba, 06123 Perugia (PG) (IT); MANCUSO, Francesca, 06123 Perugia (PG) (IT); ARATO, Iva, 06123 Perugia (PG) (IT); CECCARELLI, Veronica, 06123 Perugia (PG) (IT)
(74) Representative: Gitto, Serena
(86) International application number: PCT/IT2021/050364
(87) International publication number: WO 2022/101948

(56) References cited:
- WO-A1-2004/056370
- A. KOEBERLE ET AL: "Polyunsaturated fatty acids are incorporated into maturating male mouse germ cells by lysophosphatidic acid acyltransferase 3", THE FASEB JOURNAL, vol. 26, no. 1, 1 January 2012 (2012-01-01), & EXPERIMENTAL BIOLOGY MEETING; SAN DIEGO, CA, USA; APRIL 21 -25, 2018, pages 169 - 180, XP055248443, ISSN: 0892-6638, DOI: 10.1096/fj.11-184879
- ARATO IVA ET AL: "Effect of EPA on Neonatal Pig Sertoli Cells "In Vitro": A Possible Treatment to Help Maintain Fertility in Pre-Pubertal Boys Undergoing Treatment With Gonado-Toxic Therapies", FRONTIERS IN ENDOCRINOLOGY, vol. 12, 20 May 2021 (2021-05-20), XP055827315, DOI: 10.3389/fendo.2021.694796

## Description

The present invention relates to ω-3 polyunsaturated fatty acids for use in the prevention and treatment of male infertility in prepubertal subjects who are candidates for gonadotoxic therapies. In particular, the invention relates to ω-3 polyunsaturated fatty acids for use in the prevention and treatment of male infertility in prepubertal subjects undergoing chemotherapeutic and/or radiotherapeutic treatments for the treatment of neoplasms.

It is well-known that the recent progress achieved in the field of chemo- and radiotherapy has considerably increased the survival rate of children, adolescents and young men affected by neoplastic pathologies and severe inflammatory diseases having an autoimmune etiology, with a rate of mortality that has fallen constantly by 2.1% a year since 1975 (Siesling S et al., 2014).

However, along with the increase in the survival of patients (at least 3 out of 4) treated with the new chemotherapeutic therapies, a significant increase has been observed in sub-fertile or infertile subjects in adulthood (Siesling S et al., 2014).In fact, the chemo- and radiotherapeutic treatments used exhibit a high cytotoxicity capable of altering or completely compromising the male gonad, with a consequent reduction or loss of endocrine function and fertility (Romerius et al., 2009; Schertl 2007; Williams 2008).

In particular, the chemotherapeutics used in the treatment of malignant and non-malignant pathologies bring about an alteration of spermatogenesis and of the endocrine function of testicles (Nurmio M et al., 2009).

In Italy, on average, over 14 boys aged younger than 15 years receive a tumour diagnosis every day and more than 80% of them are successfully cured (Siesling S et al., 2014). Therefore, the infertility resulting from the therapies carried out to treat neoplasms in the prepubertal period represents a frequent consequence that occurs in adulthood as a result of the treatments carried out (Garolla A et al., 2006).

It is estimated, in fact, that about one third of patients who survive neoplasms treated in the prepubertal period will be azoospermic in adulthood and about one fifth of these subjects will be oligozoospermic (Ntemou and et al., 2019).

The data in the literature suggest that young male patients undergoing chemo- and/or radiotherapy for oncological pathologies appear to be at greater risk of infertility than women of the same age (Hamre 2012).

Whereas in adult males who undergo chemo- and/or radiotherapeutic treatments and/or are candidates for surgical interventions that potentially impair the ejaculatory function, cryopreservation of sperm from seminal fluid by now represents a reliable, well-established method that is increasingly implemented in clinical practice, this method cannot be carried out in prepubertal patients (Tournaye H et al., 2014).

In greater detail, the data in the literature show that treatment with chemotherapeutic drugs in prepubertal subjects induces a reduction in the size of the testicles, a loss of the population of germ cells, the presence of seminiferous tubules consisting solely of Sertoli cells (SCs) due to the loss of germ cells, interstitial fibrosis and thickening of the basal membrane (Hensle TW et al., 1984; Uderzo C et al.,1984).

In particular, it is known that oncological therapies in prepubertal subjects are capable of suppressing the population of testicular germline stem cells (Ad and Ap spermatogonia), which represent the cells from which spermatogenesis originates (Hussein AA et al., 2014). Chemo- and/or radiotherapeutic treatments can in fact result in a complete inhibition of spermatogenesis and ultimately in sterility due to azoospermia (Ntemou and et al., 2019; Stukenborg JB et al., 2018).

It is further known that treatment with chemotherapeutic drugs induces apoptosis of germ cells, as well as a reduction in viability (Aslani et al., 2017) and an alteration of the functionality of SCs (reduction in androgen-binding protein and transferrin) (Favareto et al., 2011). However, information about the modulation of two typical Sertolian markers, namely, the anti-Müllerian hormone (AMH) and inhibin B (Allen CM et al.,2020) is completely lacking.

The number of human spermatogonial stem cells (hSSCs), consisting of spermatogonia A (Ad and Ap), after a gradual reduction from birth to 3 years, subsequently increases until 8 years of age and, finally, after a brief decrease between 8 and 9 years, it increases considerably until 12-13 years of age. In particular, the number of SSCs appears to be unchanged from birth to 12-13 years of age, except during the period between 4 and 10 years, when the number of type Ad spermatogonia decreases slightly compared to type Ap. This period coincides with the appearance of type B spermatogonia, whose number increases progressively from 4 to 12-13 years of age.

Spermarche, that is, the period in which spermatozoa are observable in seminal fluid, occurs at 11 years of age in 1-2% of boys, at 12-13 years of age in 15-37% and at 14 years of age in 24%-69%.

SCs, which represent the only somatic cells within the seminiferous tubules, can be considered the orchestra director of spermatogenesis (Dimitriadis F et al., 2015). The contribution of SCs to spermatogenesis is based on the production of critical factors (components of the extracellular matrix, growth factors and many proteins like transferrin, clusterin and stem cell factor), necessary for the successful development of spermatogonia up to the stage of spermatozoa (Gnessi L et al., 1997; Rossi P et al., 2000).

In particular, SCs produce glial cell-derived neurotrophic factor (GDNF), which, on interacting with the RET/GFRA1 receptor complex located on the surface of undifferentiated spermatogonia, acts like a mitogenic agent, finely regulating the renewal of the residual pool of spermatogonial stem cells, the target of gonadotoxic therapies (Parekh PA et al., 2019).

In recent years a variety of evidence has been gathered regarding the possible correlation existing between the toxicity mediated by chemotherapeutic drugs and the appearance of epigenetic modifications in treated subjects (Quintanilha JCF et al., 2019; Hanf A et al., 2019).

It has already been known for some time, in fact, that DNA methylation, considered as one of the main epigenetic mechanisms, is capable of influencing male fertility (Carrell DT 2012). In particular, "DNA methylation" refers to the conversion of cytosine into 5-methylcytosine by at least 5 types of DNA methyl-transferases (DNMTs). This methylation occurs in so-called "CpG" (cytosine-phosphate-guanine) islands, i.e. in those areas of DNA in which cytosine is directly followed by a guanine in the DNA sequence. This phenomenon triggers a process (enhanced by histone lysine deacetylation) that concludes with a thickening of chromatin, which inactivates the genes involved, preventing the access of transcription factors to promotors. The final result of all this consists in a reduced gene transcription and, *de facto,* in a gene silencing (Kläver R et al., 2013). The reason for this intense DNA methylation during spermatogenesis, besides that of regulating gene expression, seems tied to the necessity of adequately organising the chromatinic structure for the growing and new needs of meiotic and spermatogenic processes. An anomalous methylation of genomic DNA, equal to about 14.4% of paternal genes, is associated with the presence of oligospermia or oligoasthenoteratozoospermia (Oakes CC et al., 2007). Furthermore, data in the literature demonstrate that the expression of GDNF is subject to mechanisms of epigenetic modification (Saracino R et al., 2020; Zhang L et al., 2019).

It is known that eicosapentaenoic acid (EPA), a fatty acid with known anti-tumour properties, is capable of decreasing the levels of DNA methylation by activating TET proteins and thereby transforming 5-methyl cytosine into 5-hydroxymethylcytosine, with a consequent re-expression of genes silenced in the methylation process (Ceccarelli et al., 2018). Furthermore, EPA is capable of inhibiting the expression and activity of the enzyme histone deacetylase and of DNA methyltransferases (HDAC1 and DNMT), responsible, respectively for deacetylation of the lysine residues of histones and for the addition of methyl groups on the CpG islands of DNA, which represent two of the most studied mechanisms in epigenetic regulation (Ceccarelli V et al., 2020).

Previous studies have demonstrated the protective effect of EPA through the inhibition of apoptosis, lipoperoxidation and the production of active radical oxygen species on cultures of mouse Sertoli cells (Hu X et al., 2018).

Furthermore, patent application WO/2004/056370 describes a fatty acid composition composed of (all the omega-3)-5,8,11,14,17-eicosapentaenoic acid (EPA) and (all the omega-3)-4,7,10,13,16,19 docosahexaenoic acid (DHA), for the production of a pharmaceutical composition or a dietary foodstuff for the prevention or treatment of male infertility. In particular, the fatty acids compositions according to the aforesaid patent application are potentially useful for the treatment and prevention of male infertility in asthenozoospermia and teratozoospermia, in the condition due to low concentrations of DHA in seminal fluid.

However, patent application WO/2004/056370 describes the aforesaid composition of EPA and DHA in the treatment and prevention of male infertility in patients affected by alterations in sperm motility (asthenozoospermia) and morphology (teratozoospermia). Therefore, the composition described in WO/2004/056370 is aimed at the treatment of subjects who have already developed spermatozoa and does not resolve the problem of infertility induced by chemotherapeutics in prepubertal individuals in whom the production of spermatozoa has not yet occurred (and who can thus not cryopreserve semen).

In the light of the foregoing, it appears evident that there is a need to provide new therapies capable of preventing and/or treating male infertility in prepubertal subjects undergoing chemo- and/or radiotherapies.

The solution according to the present invention fits into this context; it aims to provide compounds, in particular polyunsaturated fatty acids of the ω-3 series, for the prevention and treatment of male infertility, in particular azoospermia-induced infertility, due to chemo/radiotherapeutic treatments in prepubertal subjects, wherein cryopreservation cannot take place.

According to the present invention, it has now been found that in SCs of prepubertal pigs treated with cisplatin in the presence of eicosapentaenoic acid (EPA), the gene expression and protein expression of Sertolian GDNF, together with the gene expression and secretion of AMH and inhibin B, are surprisingly recovered. Therefore, according to the present invention, EPA, by acting in the recovery of GDNF and the restoration of Sertolian functionality, in an *in "vitro"* model of *SCs,* can be proposed as a therapeutic strategy in the treatment of male infertility due to chemo/radiotherapeutic treatments in prepubertal subjects, wherein cryopreservation cannot take place.

The present invention concerns polyunsaturated fatty acids of the ω-3 series for use in the treatment and prevention of male infertility in subjects undergoing chemo/radiotherapeutic treatments carried out to treat neoplasms in the prepubertal period.

The use of the ω-3 polyunsaturated fatty acids according to the present invention, in particular the use of EPA, advantageously makes it possible to induce the production of new germ cells, avoid a reduction in the number thereof or increase the number thereof in patients undergoing chemo- and/or radiotherapeutic treatments in the prepubertal period, wherein the production of spermatozoa has not yet occurred (and wherein, therefore, semen cannot be cryopreserved).

In particular, the experimental data described below show that, following treatment of SCs with chemotherapeutic drugs, it was possible to observe a significant reduction in hydroxymethylcytosine and a significant reduction in the gene expression of GDNF and of the GDNF protein compared to untreated SCs. Furthermore, a protective effect of EPA was demonstrated at a concentration of 100 µM on SCs treated at the highest dose of cisplatin (3.33 µM). This effect was demonstrated both in terms of gene and protein expression of Sertolian GDNF, and in terms of gene expression and secretion of AMH and inhibin B as specific markers of Sertolian functionality. In particular, Sertolian GDNF was surprisingly recovered compared to the control group and AMH and inhibin B reached values comparable to those of the control group.

This effect is likely to be correlated to the epigenetic action of EPA. In fact, as shown in the experimental data, the demethylating action of EPA was demonstrated when cells were treated with AZA (5-aza-2'-deoxycytidine), a known demethylating agent used in the chemotherapeutic treatment of tumour pathologies. By treating cells with the demethylating agent AZA a recovery in the expression of GDNF was obtained which was comparable to the one induced by treatment with the fatty acid EPA, although the treatment with EPA showed a greater effectiveness. Furthermore, the use of EPA as a demethylating agent advantageously makes it possible to avoid the occurrence of toxic side effects that normally accompany treatments with demethylating agents with a generalised action such as 5-AZA. In fact, EPA, only acts where it is necessary to restore a correct gene expression when the latter is modified.

It is therefore specific object of the present invention one or more ω-3 polyunsaturated fatty acids for use in the prevention and treatment of male infertility, in particular azoospermia-induced infertility, of prepubertal subjects undergoing chemotherapeutic and/or radiotherapeutic treatments, wherein said infertility is caused by said chemotherapeutic and/or radiotherapeutic treatments. Such subjects, not being able to collect semen beforehand for cryopreservation, since physiologically the production of spermatozoa has not yet begun, are in fact at high risk of infertility because of the high gonadotoxicity of such treatments.

According to the present invention, "prepubertal subjects undergoing chemotherapeutic and/or radiotherapeutic treatments" means subjects who have yet to receive the treatment, subjects who are receiving the treatment or subjects who have already received it. Therefore, said one or more ω-3 polyunsaturated fatty acids can be administered before, after or during the chemotherapeutic and/or radiotherapeutic treatment. Preferably, said one or more ω-3 polyunsaturated fatty acids are administered during or after the chemotherapeutic and/or radiotherapeutic treatment, even more preferably said one or more ω-3 polyunsaturated fatty acids are administered during the chemotherapeutic and/or radiotherapeutic treatment.

According to the present invention, said one or more polyunsaturated fatty acids can be selected from the group consisting of 20:5 ω-3 eicosapentaenoic acid, 18:3 ω-3 α-linolenic acid, 22:6 ω-3 docosahexaenoic acid and 22:5 ω-3 docosapentaenoic acid (DPA), preferably 20:5 ω-3 eicosapentaenoic acid.

Therefore, according to the present invention, mixtures of several ω-3 polyunsaturated fatty acids can be administered, for example a mixture of 20:5 ω-3 eicosapentaenoic acid and 22:6 ω-3 docosahexaenoic acid, a mixture of 20:5 ω-3 eicosapentaenoic acid and 18:3 ω-3 α-linolenic acid, a mixture of 20:5 ω-3 eicosapentaenoic acid and 22:5 ω-3 docosapentaenoic acid, a mixture of 22:6 ω-3 docosahexaenoic acid and 18:3 ω-3 α-linolenic acid, a mixture of 18:3 ω-3 α-linolenic acid and 22:5 ω-3 docosapentaenoic acid, a mixture of 22:6 ω-3 docosahexaenoic acid and 22:5 ω-3 docosapentaenoic acid, a mixture of 20:5 ω-3 eicosapentaenoic acid, 18:3 ω-3 α-linolenic acid and 22:6 ω-3 docosahexaenoic acid, a mixture of 20:5 ω-3 eicosapentaenoic acid, 18:3 ω-3 α-linolenic acid and 22:5 ω-3 docosapentaenoic acid, a mixture of 20:5 ω-3 eicosapentaenoic acid, 22:6 ω-3 docosahexaenoic acid and 22:5 ω-3 docosapentaenoic acid, a mixture of 18:3 ω-3 α-linolenic acid, 22:6 ω-3 docosahexaenoic acid and 22:5 ω-3 docosapentaenoic acid or a mixture of 20:5 ω-3 eicosapentaenoic acid, 18:3 ω-3 α-linolenic acid, 22:6 ω-3 docosahexaenoic acid and 22:5 ω-3 docosapentaenoic acid.

It is a further object of the present invention a pharmaceutical composition comprising one or more ω-3 polyunsaturated fatty acids together with one or more pharmaceutically acceptable excipients and/or adjuvants, for use in the prevention and treatment of male infertility, in particular azoospermia-induced infertility, in prepubertal subjects undergoing chemotherapeutic and/or radiotherapeutic treatments, wherein said infertility is caused by said chemotherapeutic and/or radiotherapeutic treatments.

According to the present invention, said one or more ω-3 polyunsaturated fatty acids can be selected from the group consisting of 20:5 ω-3 eicosapentaenoic acid, 18:3 ω-3 α-linolenic acid, 22:6 ω-3 docosahexaenoic acid and 22:5 ω-3 docosapentaenoic acid, preferably 20:5 ω-3 eicosapentaenoic acid.

In particular, according to the present invention, said pharmaceutical composition can comprise mixtures of several ω-3 polyunsaturated fatty acids, for example a mixture of 20:5 ω-3 eicosapentaenoic acid and 22:6 ω-3 docosahexaenoic acid, a mixture of 20:5 ω-3 eicosapentaenoic acid and 18:3 ω-3 α-linolenic acid, a mixture of 20:5 ω-3 eicosapentaenoic acid and 22:5 ω-3 docosapentaenoic acid, a mixture of 22:6 ω-3 docosahexaenoic acid and 18:3 ω-3 α-linolenic acid, a mixture of 18:3 ω-3 α-linolenic acid and 22:5 ω-3 docosapentaenoic acid, a mixture of 22:6 ω-3 docosahexaenoic acid and 22:5 ω-3 docosapentaenoic acid, a mixture of 20:5 ω-3 eicosapentaenoic acid, 18:3 ω-3 α-linolenic acid and 22:6 ω-3 docosahexaenoic acid, a mixture of 20:5 ω-3 eicosapentaenoic acid, 18:3 ω-3 α-linolenic acid and 22:5 ω-3 docosapentaenoic acid, a mixture of 20:5 ω-3 eicosapentaenoic acid, 22:6 ω-3 docosahexaenoic acid and 22:5 ω-3 docosapentaenoic acid, a mixture of 18:3 ω-3 α-linolenic acid, 22:6 ω-3 docosahexaenoic acid and 22:5 ω-3 docosapentaenoic acid or a mixture of 20:5 ω-3 eicosapentaenoic acid, 18:3 ω-3 α-linolenic acid, 22:6 ω-3 docosahexaenoic acid and 22:5 ω-3 docosapentaenoic acid.

According to the present invention, said pharmaceutical composition can further comprise one or more chemotherapeutic agents, preferably chemotherapeutic agents used in paediatric haematology-oncology. In particular, said one or more chemotherapeutic agents can be selected from the group consisting of cisplatin, 4-hydroperoxycyclophosphamide, doxorubicin, gemcitabine, methotrexate, cytosine arabinoside (ARA-C), irinotecan, vincristine, etoposide, dacarbazine, vindesine, vinblastine, vinorelbine, asparaginase, mitoxantrone and docetaxel.

Furthermore, according to the present invention, said pharmaceutical composition can further comprise one or more antioxidant agents. In particular, said one or more antioxidant agents can be selected from the group consisting of vitamin E, vitamin C, beta-carotene, N-acetylcysteine, alpha-lipoic acid, carnitine, folic acid, lycopene, selenium, coenzyme Q-10 and zinc.

The present invention further relates to a combination of one or more ω-3 polyunsaturated fatty acids with one or more agents selected from the group consisting of chemotherapeutic agents and antioxidant agents, for separate or sequential use in the prevention and treatment of male infertility, in particular azoospermia-induced infertility, in prepubertal subjects undergoing chemotherapeutic and/or radiotherapeutic treatments, wherein said infertility is caused by said chemotherapeutic and/or radiotherapeutic treatments.

According to the present invention, said one or more chemotherapeutic agents can be selected from the group consisting of cisplatin, 4-hydroperoxycyclophosphamide, doxorubicin, gemcitabine, methotrexate, cytosine arabinoside (ARA-C), irinotecan, vincristine, etoposide, dacarbazine, vindesine, vinblastine, vinorelbine, asparaginase, mitoxantrone and docetaxel.

Furthermore, according to the present invention, said one or more antioxidant agents can be selected from the group consisting of vitamin E, vitamin C, beta-carotene, N-acetylcysteine, alpha-lipoic acid, carnitine, folic acid, lycopene, selenium, coenzyme Q-10 and zinc.

According to the present invention, "separate use" means the administration, at the same time, of the compounds of the combination according to the invention in distinct pharmaceutical forms. "Sequential use" means the sequential administration of the compounds of the combination according to the invention, each in a distinct pharmaceutical form.

The present invention will now be described by an illustrative, but not limitative, way, according to a preferred embodiment thereof, with particular reference to the examples and figures of the appended drawings, wherein:

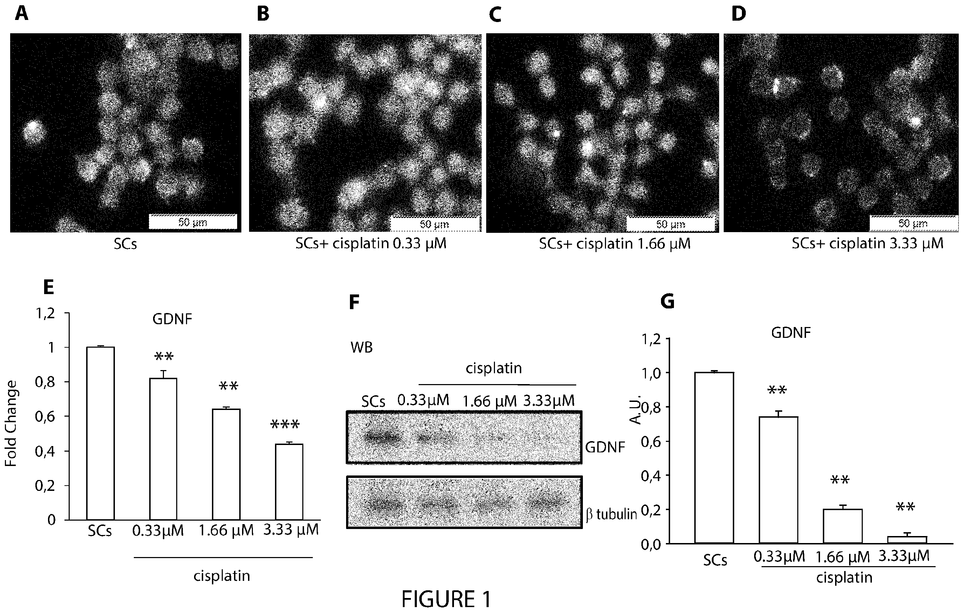

- **Figure 1** shows the treatment with cisplatin: 5hmC was evaluated by IF (A-D) and GDNF by real-time PCR (E), WB and densitometric analysis (F, G), both in the control group and after treatment with cisplatin 0.333, 1.66 and 3.33 µM. It is possible to observe a progressive reduction in immunolabelling for 5hmC (white plots) with increases in the dose of the chemotherapeutic and a significant reduction in the gene and protein expression of GDNF with increases in the dose of the chemotherapeutic. The data represent the mean ± S.E.M. (**p <0.01 and ***p <0.001 compared to untreated SCs) of three independent experiments, each carried out in triplicate.

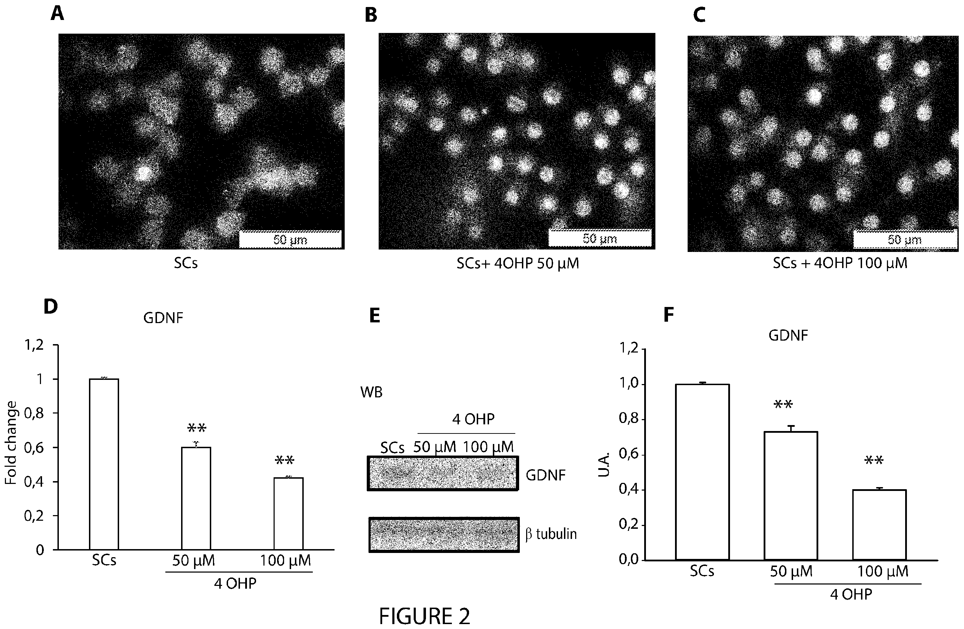

- **Figure 2** shows the treatment with 4OHP: 5hmC was evaluated by IF (A-D) and GDNF by real-time PCR (E), WB and densitometric analysis (F, G), both in the control group and after treatment with 40HP 50 and 100 µM. It is possible to observe a progressive reduction in immunolabelling for 5hmC (white plots) with increases in the dose of the chemotherapeutic and a significant reduction in the gene and protein expression of GDNF with increases in the dose of the chemotherapeutic. The data represent the mean ± S.E.M. (**p <0.01 and ***p <0.001 compared to untreated SCs) of three independent experiments, each carried out in triplicate.

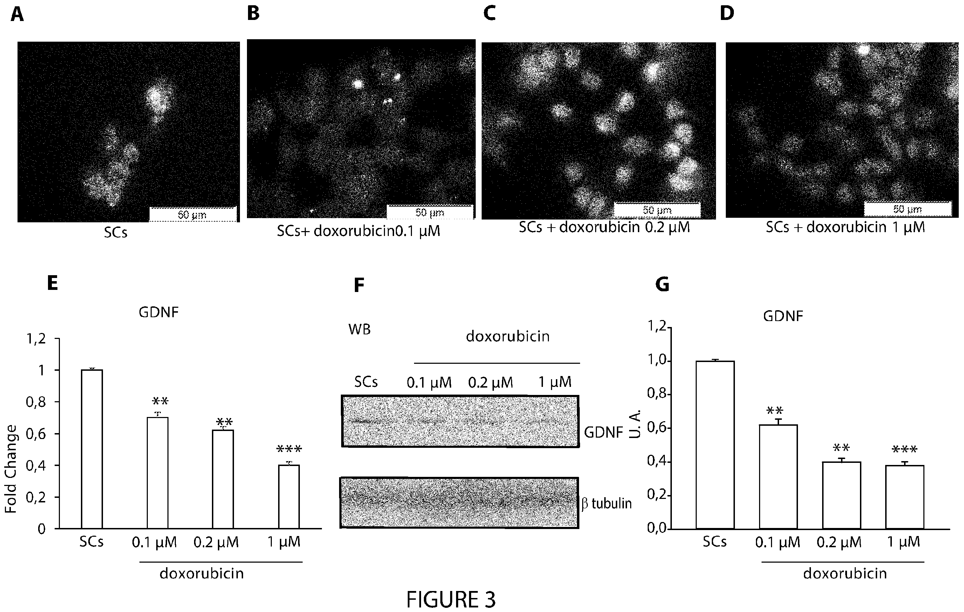

- **Figure 3** shows the treatment with doxorubicin: 5hmC was evaluated by IF (A-D) and GDNF by real-time PCR (E), WB and densitometric analysis (F, G), both in the control group and after treatment with doxorubicin 0.1, 0.2 and 1 µM. It is possible to observe a progressive reduction in immunolabelling for 5hmC (white plots) with increases in the dose of the chemotherapeutic and a significant reduction in the gene and protein expression of GDNF with increases in the dose of the chemotherapeutic. The data represent the mean ± S.E.M. (**p <0.01 and ***p <0.001 compared to untreated SCs) of three independent experiments, each carried out in triplicate.

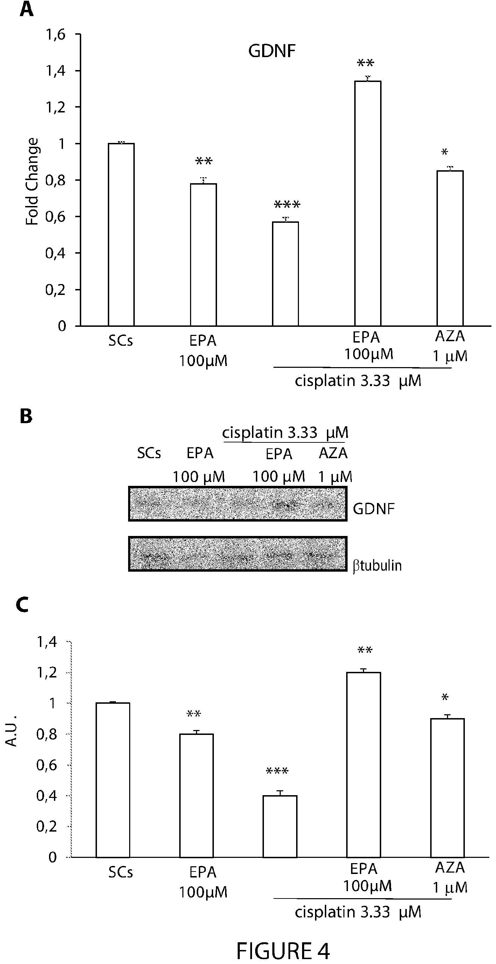

- **Figure 4** shows the protective effect of EPA 100µM on the gene expression of GDNF (A) and protein expression after treatment with cisplatin 3,33 µM by means of WB (B) and densitometric analysis (C). The data represent the mean ± S.E.M. (*p <0.05, **p <0.01, and ***p <0.001 compared to untreated SCs) of three independent experiments, each carried out in triplicate.

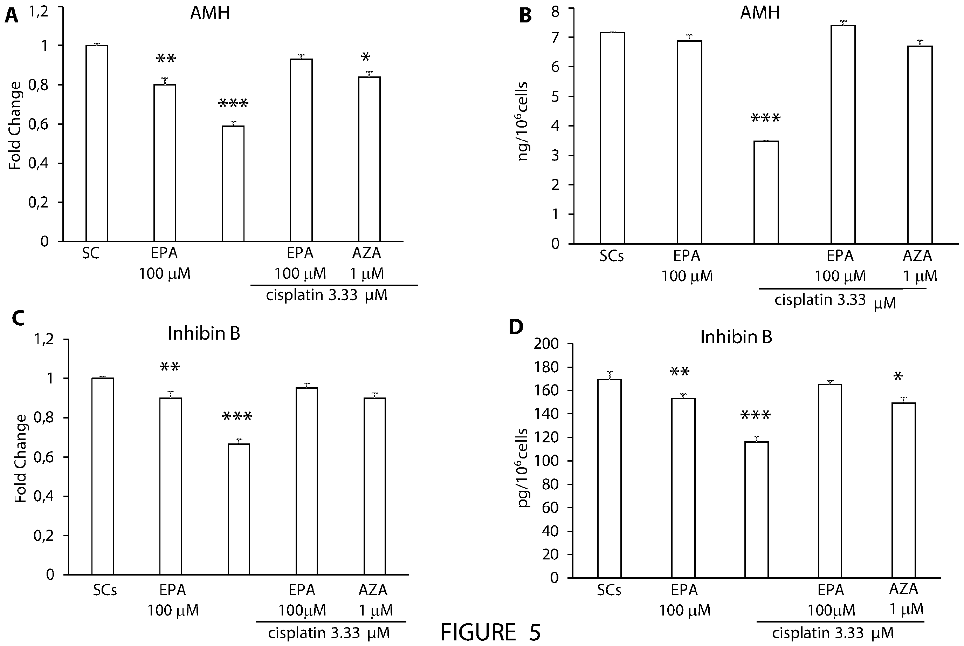

- **Figure 5** shows the protective effect of EPA in terms of gene expression and secretion of AMH (A, B) and inhibin B (C, D), as specific markers of Sertolian functionality, after treatment with cisplatin 3.33µM plus EPA 100µM. The data represent the mean ± S.E.M. (**p <0.01 and ***p <0.001 compared to untreated SCs) of three independent experiments, each carried out in triplicate.

### EXAMPLE 1. Study on the functionality of Sertoli cells (SCs) of prepubertal pigs treated with chemotherapeutic drugs and eicosapentaenoic acid (EPA).

### Materials and methods

### Isolation and purity of the SCs

The animal studies were conducted in accordance with national guidelines (approved Italian animal protection certification A-3143-01) and guidelines of the Committee for the Care and Use of Animals of the University of Perugia. The experimental protocols were approved by the University of Perugia. The newborn prepubertal "Large White" pigs (Farm "Trequattrini", S. Elena di Marsciano, PG), aged 7-15 days, were used as SC donors. The SCs were isolated in accordance with previously established methods, modified in our laboratory (Luca et al., 2015).

Immunostaining for AMH, a specific and unique marker of prepubertal SCs, 3-βHSD, specific for Leydig cells and ASMA, specific for peritubular cells was performed in accordance with previously established methods, with slight modifications (Luca et al., 2015). After the isolation/purification procedure had been completed, the cultures were composed of highly purified SCs (95%), as indicated by the immunostaining for AMH, with an extremely low percentage of "contaminating cells" (5%), (Luca et al., 2015).

### Culture of SCs and treatment with chemotherapeutic drugs

The SCs were maintained at 37°C and 5% CO2 in HAMF-12 (Euroclone, MI, Italy) supplemented with 0.166 nM of retinoic acid (Sigma-Aldrich, St. Louis, MO, USA) and 5 mL/500ml of ITS (Becton Dickinson cat. no. 354352, NJ, USA) in the absence (untreated) or presence of chemotherapeutic drugs for 48 hours plus 24 of recovery at the following concentrations:
- Cisplatin 0.33 µM, 1.66 µM and 3.33 µM;
- 4-hydroperoxycyclophosphamide (40HP) 50 and 100 µM;
- Doxorubicin 0.1 µM, 0.2 µM and 1 µM;
- cisplatin 3.33 µM plus EPA 100 µM;
- cisplatin 3.33 µM plus 5-aza-2'-deoxy-cytidine (5 AZA) 1 µM.

The SCs were maintained at 37°C and 5% CO2 in HAMF-12 (Euroclone, MI, Italia) supplemented with 0.166 nM of retinoic acid (Sigma-Aldrich, St. Louis, MO, USA) and 5 mL/500ml of ITS (Becton Dickinson cat. no. 354352, NJ, USA) in the absence (untreated) or presence of cisplatin 0.5 and 1 µg/ml for 48 hours plus 24 of recovery plus 100µM) EPA.

### Immunofluorescence analysis

The untreated SCs and SCs treated with chemotherapeutic drugs (plus EPA or without EPA), were made to grow on round slides pre-treated with gelatine (Sigma-Aldrich). At the end of the treatment, the SCs were fixed for 30 min in 4% paraformaldehyde in phosphate buffer (PBS). Prior to immunolabelling, the cells were subjected to permeabilization (PBS, 0.2% Triton X) for 5 min at room temperature, blocked in 0.5% bovine serum albumin (BSA; Sigma-Aldrich, St. Louis, MO) in PBS for 10 min, and then exposed to the primary anti-5hmC antibody (Santa Cruz Biotechnology, 1:100 in PBS) overnight at 4° C. The cells were washed three times in PBS for 5 min and then exposed to the secondary Alexa-488 labelled antibody (1:500 in PBS). The cells were counterstained with 4',6-diamidino-2-phenylindole (DAPI) for 1 min after treatment with RNase (10 mg/mL per 10 min.). The negative control for the immunolabelling consisted in skipping the labelling with the primary antibody and passing directly to the secondary antibody. The slides were then mounted with ProLong^{®} Gold Antifade (ThermoFisher) and the images were acquired with an Olympus BX-41 equipped with Cell-F image processing software (Olympus).

### Real-time PCR analysis

Total RNA was isolated from the monolayers of untreated SCs and SCs treated with chemotherapeutic drugs (plus EPA or without EPA) by means of an RNA purification kit (Versagene RNA Cell Kit, Gentra Systems, Minneapolis, MN) and quantified by reading the optical density at 260 nm. Subsequently, 1 µg of total RNA was subjected to RT in a final volume of 20 µl by means of the Maxima First Strand cDNA Synthesis kit (Thermo Scientific, MA, USA). Real-time PCR was performed using 16 ng of cDNA, prepared from the RT reaction, and SYBR Green (Stratagene, Amsterdam, Netherlands). Real-time PCR was performed with an Mx3000P cycler (Stratagene) using FAM for detection and ROX as the reference dye. The level of mRNA for each sample was normalised with respect to the mRNA of β-actin and expressed as a change compared to the level of the untreated control cells.

### Western Blotting analysis

The SCs were cultured at 37°C and 5% CO₂ in HAMF-12 (Euroclone, MI, Italy) supplemented with 0.166 nM of retinoic acid (Sigma-Aldrich, St. Louis, MO, USA) and 5 mL/500ml of ITS (Becton Dickinson cat. no. 354352, NJ, USA) in the absence (untreated) or presence of cisplatin 0.5 and 1 µg/ml for 48 hours plus 24 of recovery and with the addition of 100µM EPA. 70µg of proteins obtained from the total cell lysate were subjected to SDS gel electrophoresis, then electroblotted on a nitrocellulose membrane (SchleicherandSchuell, Keene, NH, USA) and labelled with an anti-5hmC antibody (Santa Cruz Biotechnology). The immunoreactive bands were visualised by means of an ECL assay (Amersham Pharmacia Biotech, Little Chalfont, UK). The anti-β-tubulin antibody (Sigma-Aldrich) was used as a normaliser. The images were acquired by means of the VersaDoc Imaging System (Bio-Rad Hercules, CA, USA), and the respective signals were quantified using Quantity One software (Bio-Rad) 2.8.

### Analysis of the secretion of Inhibin B and AMH

At the end of the treatment with the chemotherapeutic drugs, aliquots of the supernatants were collected from cultures of untreated SCs and SCs treated with the chemotherapeutic drugs (plus EPA or without EPA) and stored at -20°C for the assessment of inhibin B and AMH secretion by ELISA (Inhibin B Gen II ELISA kit, Beckman Coulter, Webster, TX, U.S.A., within the tests CV=2.81%; between the tests CV=4.33%), (AMH Gen II ELISA kit, Beckman Coulter, Webster, TX, U.S.A., within the tests CV=3.89%; between the tests CV=5.77%), as previously described (Luca et al., 2015).

### Statistical analysis

The results shown in the figures were the mean ± S.E.M of three independent experiments, each carried out in triplicate. The statistical analysis was performed by means of a paired Student T-test.

### Results

The data obtained demonstrated (Fig.1), after treatment of the SCs with Cisplatin at the concentrations of 0.33 µM, 1.66 µM and 3.33 µM:
- a significant reduction in 5-hydroxymethylcytosine (5hmC) (oxidation product of 5-methylcytosine, in turn generated by DNMTs on CpG islands, and whose presence is associated with an active transcription of DNA), assessed by means of immunofluorescence analysis;
- a significant reduction in the gene and protein expression of GDNF, assessed by means of Real-Time PCR and western blotting analysis.
   Fig. 2 shows, after treatment of the SCs with 4-hydroperoxycyclophosphamide (40HP) (active metabolite of cyclophosphamide) at the concentrations of 50 and 100 µM:
- a significant reduction in 5-hydroxymethylcytosine (5hmC), assessed by means of immunofluorescence analysis;
- a significant reduction in the gene and protein expression of GDNF, assessed by means of real-time PCR and western blotting analysis. Fig. 3 shows, after treatment of the SCs with doxorubicin at the concentrations of 0.1 µM, 0.2 µM and 1 µM
   - a significant reduction in 5-hydroxymethylcytosine (5hmC), assessed by means of immunofluorescence analysis;
   - a significant reduction in the gene and protein expression of GDNF, assessed by means of real-time PCR and western blotting analysis.

Fig. 4 shows a recovery both of the gene and protein expression of Sertolian GDNF after treatment of the SCs with cisplatin 3.33 µM in the presence of 100µM) EPA.

Fig. 5 shows a recovery both in terms of gene expression and of secretion of AMH and inhibin B, as specific markers of Sertolian functionality, after treatment of the SCs with cisplatin3.33 µM, in the presence of 100µM) EPA.

This effect is likely to be correlated to the epigenetic action of EPA, whose demethylating action has been demonstrated (Ceccarelli V et al., 2018). In fact, by treating the cells with AZA (5-aza-2'-deoxycytidine), a known demethylating agent used in the chemotherapeutic treatment of tumour pathologies, one obtains a recovery comparable to that induced by treatment with the fatty acid.

### References

Allen CM, Lopes F, Mitchell RT, Spears N . Comparative gonadotoxicity of the chemotherapy drugs cisplatin and carboplatin on prepubertal mouse gonads. Mol Hum Reprod. 2020; 26:129-140.
Aslani F, Sebastian T, Keidel M, Fröhlich S, Elsässer H, Schuppe H, Klug J, Mahavadi P, Fijak M, Bergmann M, Meinhardt A, Bhushanet S. Resistance to apoptosis and autophagy leads to enhanced survival in Sertoli cells. Molecular Human Reproduction 2017; 23:370-380.
Carrell DT. Epigenetics of the male gamete. Fertil Steril. 2012; 97:267-274.
Ceccarelli V, Valentini V, Ronchetti S, Cannarile L, Billi M, Riccardi C, Ottini L, Talesa VN, Grignani F, Vecchini A. Eicosapentaenoic acid induces DNA demethylation in carcinoma cells through a TET1-dependent mechanism. FASEB J. 2018; 14:fj201800245R.
Ceccarelli V, Ronchetti S, Marchetti MC, Calvitti M, Riccardi C, Grignani F, Vecchini A. 2020. Molecular mechanisms underlying eicosapentaenoic acid inhibition of HDAC1 and DNMT expression and activity in carcinoma cells. Biochim Biophys Acta Gene Regul Mech. 2020; 1863:194481.
Dimitriadis F, Tsiampali C, Chaliasos N, Tsounapi P, Takenaka A, Sofikitis N. The Sertoli cell as the orchestra conductor of spermatogenesis: spermatogenic cells dance to the tune of testosterone Hormones 2015; 14:479-503.
Favareto APA, Fernandez CDB, Fossato da Silva DA, Anselmo-Franci JA, De Grava Kempinas W. Persistent Impairment of Testicular Histology and Sperm Motility in Adult Rats Treated with Cisplatin at Peri-Puberty. Basic & Clinical Pharmacology & Toxicology. 2011, 109:85-96.
Garolla A, Pizzato C, Ferlin A, Carli MO, Selice R, Foresta C. Progress in the development of childhood cancer therapy. Reprod Toxicol 2006; 22:126-132.
Gnessi L, Fabbri A, Spera G. Gonadal peptides as mediators of development and functional control of the testis: an integrated system with hormones and local environment. Endocr Rev 1997; 18:541- 609.
Hamre H, Kiserud CE, Ruud E, Thorsby PM, Fosså SD. Gonadal function and parenthood 20 years after treatment for childhood lymphoma: a cross-sectional study. Pediatr Blood Cancer. 2012; 59:271-277.
Hanf A, Oelze M, Manea A, Li H, Münzel T, Daiber A. The anticancer drug doxorubicin induces substantial epigenetic changes in cultured cardiomyocytes. Chem Biol Interact. 2019; 313:108834.
Hensle TW, Burbige KA, Shepard BR, Marboe CC, Blanc WA, Wigger JH. Chemotherapy and its effect on testicular morphology in children. Journal of Urology 1984; 131:1142-1144.
Hu X, Ge X, Liang W, Shao Y, Jing J, Wang C. Effects of saturated palmitic acid and omega-3 polyunsaturated fatty acids on Sertoli cell apoptosis. Ournal. Systems Biology in Reproductive Medicine 2018; 64:368-380.
Hussein AA, Tran ND, Smith JF. Fertility preservation for boys and adolescents facing sterilizing medical therapy. Transl Androl Urol. 2014; 3:382-390.
Kläver R, Tüttelmann F, Bleiziffer A, Haaf T, Kliesch S, Gromoll J. DNA methylation in spermatozoa as a prospective marker in andrology. Andrology 2013; 1:731-740.
Luca G, Mancuso F, Calvitti M, Arato I, Falabella G, Bufalari A, De Monte V, Tresoldi E, Nastruzzi C, Basta G, Fallarino F, Lilli C, Bellucci C, Baroni T, Aglietti MC, Giovagnoli S, Cameron DF, Bodo M,
Calafiore R. Long-term stability, functional competence, and safety of microencapsulated specific pathogen-free neonatal porcine Sertoli cells: a potential product for cell transplant therapy. Xenotransplantation 2015; 22:273-83.
Ntemou E, Alexandri C, Lybaert P, Goossens E, Demeestere I. Oncofertility: Pharmacological Protection and Immature Testicular Tissue (ITT)-Based Strategies for Prepubertal and Adolescent Male Cancer Patients. Int J Mol Sci. 2019; 20:5223.
Nurmio M, Keros V, Lahteenmaki P, Salmi T, Kallajoki M, Jahnukainen K. Effect of childhood acute lymphoblastic leukemia therapy on spermatogonia populations and future fertility. J Clin Endocrinol Metab 2009; 94:2119-2122.
Oakes CC, La Salle S, Smiraglia DJ, Robaire B, Trasler JM. A Unique configuration of genome-wide DNA methylation pattern in the testis. Proc Nat Acad Sci USA 2007; 104:228-233.
Parekh PA, Garcia TX, Marie-Claude Hofmann MC. Regulation of GDNF expression in Sertoli cells. Reproduction 2019; 157:R95-R107.
Quintanilha JCF, Saavedra KF, Visacri MB, Moriel P, Salazar LA. Role of epigenetic mechanisms in cisplatin-induced toxicity. Crit Rev Oncol Hematol. 2019; 137:131-142.
Romerius P, Stahl O, Moell C, Relander T, Cavallin-Stahl E, Wiebe T, Lundberg Giwercman Y, Giwercman A. Hypogonadism risk in men treated for childhood cancer. J Clin Endocrinol Metab 2009; 94:4180-4186.
Rossi P, Sette C, Dolci S, Geremia R. Role of c-kit in mammalian spermatogenesis. J Endocrinol Invest 2000; 23:609-615.
Saracino R, Capponi C, Di Persio S, Boitani C, Masciarelli S, Fazi F, Fera S, Vicini E.
Regulation of Gdnf expression by retinoic acid in Sertoli cells. Mol Reprod Dev. 2020; 87:419-429.
Schertl S, Hartmann RW, Batzl-Hartmann C, Spruss T, Maucher A, von Angerer E, Schiller CD, Schneider MR, Gust R, Schönenberger H. Platinum(II) complexes interfering with testicular steroid biosynthesis: drugs for the therapy of advanced or recurrent prostate cancers? Preclinical studies. J Cancer Res Clin Oncol. 2007; 133:153-167.
Siesling S, Louwman WJ, Kwast A, van den Hurk C, O'Callaghan M, Rosso S, Zanetti R, Storm H, Comber H, Steliarova-Foucher E, Coebergh JW. Uses of cancer registries for public health and clinical research in Europe: results of the European Network of Cancer Registries survey among 161 population based cancer registries during 2010-2012. Eur J Cancer 2015; 51:1039-1049.
Stukenborg J -B, Alves-Lopes J P, Kurek M, Albalushi H, Reda A, Keros V, Töhönen V, Bjarnason R, Romerius P, Sundin M, Norén Nyström U, Langenskiöld C, Vogt H, Henningsohn L, Mitchell R T , Söder O, Petersen C, Jahnukainen K. Spermatogonial quantity in human prepubertal testicular tissue collected for fertility preservation prior to potentially sterilizing therapy. Human Reproduction 2018; 33:1677-1683.
Tournaye H, Dohle GR, Barratt CL. Fertility preservation in men with cancer. Lancet 2014; 384:1295-1301.
Uderzo C, Locasciulli A, Marzorati R, Adamoli L, Di Natale B, Nizzoli G, Cazzaniga M, Masera G. Correlation of gonadal function with histology of testicular biopsies at treatment discontinuation in childhood acute leukemia. Medical and Pediatric Oncology 1984; 12:97-100.
Williams D, Crofton PM, Levitt G. Does ifosfamide affect gonadal function? Pediatr Blood Cancer. 2008; 50:347-351.
Zhang L, Wang D, Han X, Tang F, Gao D. Mechanism of methylation and acetylation of high GDNF transcription in glioma cells: A review. Heliyon. 2019; 5:e01951.

## Claims

1. One or more ω-3 polyunsaturated fatty acids for use in the prevention and treatment of male infertility in prepubertal subjects undergoing chemotherapeutic and/or radiotherapeutic treatments.

2. One or more ω-3 polyunsaturated fatty acids for use according to claim 1, wherein said one or more polyunsaturated fatty acids are selected from the group consisting of 20:5 ω-3 eicosapentaenoic acid, 18:3 ω-3 α-linolenic acid, 22:6 ω-3 docosahexaenoic acid and 22:5 ω-3 docosapentaenoic acid, preferably 20:5 ω-3 eicosapentaenoic acid.

3. Pharmaceutical composition comprising one or more ω-3 polyunsaturated fatty acids together with one or more pharmaceutically acceptable excipients and/or adjuvants, for use in the prevention and treatment of male infertility in prepubertal subjects undergoing chemotherapeutic and/or radiotherapeutic treatments.

4. Pharmaceutical composition for use according to claim 3, wherein said one or more ω-3 polyunsaturated fatty acids are selected from the group consisting in 20:5 ω-3 eicosapentaenoic acid, 18:3 ω-3 α-linolenic acid, 22:6 ω-3 docosahexaenoic acid and 22:5 ω-3 docosapentaenoic acid, preferably 20:5 ω-3 eicosapentaenoic acid.

5. Pharmaceutical composition for use according to any one of claims 3-4, said pharmaceutical composition further comprising one or more chemotherapeutic agents.

6. Pharmaceutical composition for use according to claim 5, wherein said one or more chemotherapeutic agents are selected from the group consisting of cisplatin, 4-hydroperoxycyclophosphamide, doxorubicin, gemcitabine, methotrexate, cytosine arabinoside, irinotecan, vincristine, etoposide, dacarbazine, vindesine, vinblastine, vinorelbine, asparaginase, mitoxantrone and docetaxel.

7. Pharmaceutical composition for use according to any one of claims 3-6, said pharmaceutical composition further comprising one or more antioxidant agents.

8. Pharmaceutical composition for use according to claim 7, wherein said one or more antioxidant agents are selected from the group consisting of vitamin E, vitamin C, beta-carotene, N-acetylcysteine, alpha-lipoic acid, carnitine, folic acid, lycopene, selenium, coenzyme Q-10 and zinc.

9. Combination of one or more ω-3 polyunsaturated fatty acids with one or more agents selected from the group consisting of chemotherapeutic agents and antioxidant agents, for separate or sequential use in the prevention and treatment of male infertility in prepubertal subjects undergoing chemotherapeutic and/or radiotherapeutic treatments.

10. Combination for use according to claim 9, wherein said one or more chemotherapeutic agents are selected from the group consisting of cisplatin, 4-hydroperoxycyclophosphamide, doxorubicin, gemcitabine, methotrexate, cytosine arabinoside, irinotecan, vincristine, etoposide, dacarbazine, vindesine, vinblastine, vinorelbine, asparaginase, mitoxantrone and docetaxel.

11. Combination for use according to any one of claims 9-10, wherein said one or more antioxidant agents are selected from the group consisting of vitamin E, vitamin C, beta-carotene, N-acetylcysteine, alpha-lipoic acid, carnitine, folic acid, lycopene, selenium, coenzyme Q-10 and zinc.

## Patentansprüche

1. Eine oder mehrere mehrfach ungesättigte ω-3-Fettsäuren zur Verwendung bei der Vorbeugung und Behandlung von männlicher Unfruchtbarkeit bei präpubertären Probanden, die sich einer chemotherapeutischen und/oder radiotherapeutischen Behandlung unterziehen.

2. Eine oder mehrere mehrfach ungesättigte ω-3 Fettsäuren zur Verwendung gemäß Anspruch 1, wobei die eine oder mehreren mehrfach ungesättigten Fettsäuren aus der Gruppe ausgewählt sind, die aus 20:5 ω-3 Eicosapentaensäure, 18:3 ω-3 α-Linolensäure, 22:6 ω-3 Docosahexaensäure und 22:5 ω-3 Docosapentaensäure, vorzugsweise 20:5 ω-3 Eicosapentaensäure, besteht.

3. Pharmazeutische Zusammensetzung, die eine oder mehrere mehrfach ungesättigte ω-3 Fettsäuren zusammen mit einem oder mehreren pharmazeutisch annehmbaren Hilfsstoffen und/oder Adjuvantien enthält, zur Verwendung bei der Vorbeugung und Behandlung von männlicher Unfruchtbarkeit bei präpubertären Probanden, die sich einer chemotherapeutischen und/oder radiotherapeutischen Behandlung unterziehen.

4. Pharmazeutische Zusammensetzung zur Verwendung nach Anspruch 3, wobei die eine oder mehreren ω-3 mehrfach ungesättigten Fettsäuren aus der Gruppe ausgewählt sind, die aus 20:5 ω-3 Eicosapentaensäure, 18:3 ω-3 α-Linolensäure, 22:6 ω-3 Docosahexaensäure und 22:5 ω-3 Docosapentaensäure, vorzugsweise 20:5 ω-3 Eicosapentaensäure, besteht.

5. Pharmazeutische Zusammensetzung zur Verwendung nach einem der Ansprüche 3-4, wobei die pharmazeutische Zusammensetzung außerdem ein oder mehrere chemotherapeutische Mittel enthält.

6. Pharmazeutische Zusammensetzung zur Verwendung nach Anspruch 5, wobei das eine oder die mehreren chemotherapeutischen Mittel ausgewählt sind aus der Gruppe bestehend aus Cisplatin, 4-Hydroperoxycyclophosphamid, Doxorubicin, Gemcitabin, Methotrexat, Cytosinarabinosid, Irinotecan, Vincristin, Etoposid, Dacarbazin, Vindesin, Vinblastin, Vinorelbin, Asparaginase, Mitoxantron und Docetaxel.

7. Pharmazeutische Zusammensetzung zur Verwendung nach einem der Ansprüche 3-6, wobei die pharmazeutische Zusammensetzung außerdem ein oder mehrere Antioxidationsmittel enthält.

8. Pharmazeutische Zusammensetzung zur Verwendung nach Anspruch 7, wobei das
ein oder mehrere Antioxidationsmittel ausgewählt sind aus der Gruppe bestehend aus Vitamin E, Vitamin C, Beta-Carotin, N-Acetylcystein, Alpha-Liponsäure, Carnitin, Folsäure, Lycopin, Selen, Coenzym Q-10 und Zink.

9. Kombination einer oder mehrerer mehrfach ungesättigter ω-3 Fettsäuren mit einem oder mehreren Wirkstoffen, ausgewählt aus der Gruppe bestehend aus Chemotherapeutika und Antioxidantien, zur getrennten oder aufeinanderfolgenden Verwendung bei der Vorbeugung und Behandlung männlicher Unfruchtbarkeit bei präpubertären Patienten, die sich einer chemotherapeutischen und/oder radiotherapeutischen Behandlung unterziehen.

10. Kombination zur Verwendung nach Anspruch 9, wobei das eine oder die mehreren chemotherapeutischen Mittel ausgewählt sind aus der Gruppe bestehend aus Cisplatin, 4-Hydroperoxycyclophosphamid, Doxorubicin, Gemcitabin, Methotrexat, Cytosinarabinosid, Irinotecan, Vincristin, Etoposid, Dacarbazin, Vindesin, Vinblastin, Vinorelbin, Asparaginase, Mitoxantron und Docetaxel.

11. Kombination zur Verwendung nach einem der Ansprüche 9-10, wobei das eine oder die mehreren antioxidativen Mittel ausgewählt sind aus der Gruppe bestehend aus Vitamin E, Vitamin C, Beta-Carotin, N-Acetylcystein, Alpha-Liponsäure, Carnitin, Folsäure, Lycopin, Selen, Coenzym Q-10 und Zink.

## Revendications

1. Un ou plusieurs acides gras polyinsaturés ω-3 pour la prévention et le traitement de l'infertilité masculine chez les sujets prépubères soumis à des traitements chimiothérapeutiques et/ou radiothérapeutiques.

2. Un ou plusieurs acides gras polyinsaturés ω-3 pour l'utilisation selon la revendication 1, dans lequel ce ou ces acides gras polyinsaturés sont choisis dans le groupe constitué de l'acide 20:5 ω-3 eicosapentaénoïque, de l'acide 18:3 ω-3 α-linolénique, de l'acide 22:6 ω-3 docosahexaénoïque et de l'acide 22:5 ω-3 docosapentaénoïque, de préférence l'acide 20:5 ω-3 eicosapentaénoïque.

3. Composition pharmaceutique comprenant un ou plusieurs acides gras polyinsaturés ω-3 avec un ou plusieurs excipients et/ou adjuvants pharmaceutiquement acceptables, pour utilisation dans la prévention et le traitement de l'infertilité masculine chez les sujets prépubères soumis à des traitements chimiothérapeutiques et/ou radiothérapeutiques.

4. Composition pharmaceutique pour utilisation selon la revendication 3, dans laquelle ledit ou lesdits acides gras polyinsaturés ω-3 sont choisis dans le groupe constitué par l'acide 20:5 ω-3 eicosapentaénoïque, l'acide 18:3 ω-3 α-linolénique, l'acide 22:6 ω-3 docosahexaénoïque et l'acide 22:5 ω-3 docosapentaénoïque, de préférence l'acide 20:5 ω-3 eicosapentaénoïque.

5. Composition pharmaceutique utilisée selon l'une des revendications 3-4, ladite composition pharmaceutique comprenant en outre un ou plusieurs agents chimiothérapeutiques.

6. Composition pharmaceutique pour utilisation selon la revendication 5, dans laquelle ledit ou plusieurs agents chimiothérapeutiques sont choisis dans le groupe consistant en cisplatine, 4-hydroperoxycyclophosphamide, doxorubicine, gemcitabine, méthotrexate, cytosine arabinoside, irinotécan, vincristine, étoposide, dacarbazine, vindésine, vinblastine, vinorelbine, asparaginase, mitoxantrone et docétaxel.

7. Composition pharmaceutique utilisée selon l'une des revendications 3-6, ladite composition pharmaceutique comprenant en outre un ou plusieurs agents antioxydants.

8. Composition pharmaceutique selon la revendication 7, dans laquelle ledit
un ou plusieurs agents antioxydants sont choisis dans le groupe constitué de la vitamine E, de la vitamine C, du bêta-carotène, de la N-acétylcystéine, de l'acide alpha-lipoïque, de la carnitine, de l'acide folique, du lycopène, du sélénium, de la coenzyme Q-10 et du zinc.

9. Combinaison d'un ou plusieurs acides gras polyinsaturés ω-3 avec un ou plusieurs agents choisis dans le groupe des agents chimiothérapeutiques et des agents antioxydants, pour une utilisation séparée ou séquentielle dans la prévention et le traitement de la stérilité masculine chez les sujets prépubères soumis à des traitements chimiothérapeutiques et/ou radiothérapeutiques.

10. Combinaison selon la revendication 9, dans laquelle un ou plusieurs agents chimiothérapeutiques sont choisis dans le groupe constitué par le cisplatine, le 4-hydroperoxycyclophosphamide, la doxorubicine, la gemcitabine, le méthotrexate, la cytosine arabinoside, l'irinotécan, la vincristine, l'étoposide, la dacarbazine, la vindésine, la vinblastine, la vinorelbine, l'asparaginase, la mitoxantrone et le docétaxel.

11. Combinaison utilisable selon l'une des revendications 9-10, dans laquelle un ou plusieurs agents antioxydants sont choisis dans le groupe constitué par la vitamine E, la vitamine C, le bêta-carotène, la N-acétylcystéine, l'acide alpha-lipoïque, la carnitine, l'acide folique, le lycopène, le sélénium, la coenzyme Q-10 et le zinc.
